# EUROPEAN PATENT SPECIFICATION

(11) **EP 3 366 319 B1**
(45) Date of publication and mention of the grant of the patent: **26.08.2020**
(21) Application number: 17020409.3
(22) Date of filing: 07.09.2017
(51) Int. Cl.: A61L 27/18, B33Y 80/00, B33Y 70/00, A61L 27/12, A61K 33/08, A61L 27/46, A61L 27/56, A61K 33/42

(54) **THREE-DIMENSIONAL STRUCTURES BASED ON HYDROXYAPATITE AND POLYURETHANE DIOL OBTAINED THROUGH 3D PRINTING TECHNOLOGY**
DREIDIMENSIONALE STRUKTUREN AUF BASIS VON HYDROXYAPATIT UND POLYURETHANDIOL, DIE DURCH EINE 3D-DRUCK-TECHNOLOGIE HERGESTELLT WERDEN
STRUCTURES TRIDIMENSIONNELLES À BASE D' HYDROXYAPATITE ET DE POLYURÉTHANE DIOL OBTENUES À L'AIDE DE LA TECHNOLOGIE D'IMPRESSION 3D

(30) Priority: 23.02.2017 RO 102
(43) Date of publication of application: 29.08.2018
(73) Proprietor: National Research-Development Institute for Non-ferrous and Rare Metals (IMNR), 077145 Pantelimon (RO); University of Medicine and Pharmacy "Carol Davila", 020022 Bucharest (RO); SC SITEX 45 SRL, 023709 Bucharest (RO)
(72) Inventor: Popescu, Laura Madalina, 062088 Bucuresti (RO); Piticescu, Roxana Mioara, 011137 Bucuresti (RO); Motoc, Adrian Mihail, 062083 Bucuresti (RO); Voinea, Liliana Mary, 061081 Bucuresti (RO); Gradinaru (Istrate), Sînziana Luminita, 051192 Bucuresti (RO); Ulieru, Dumitru, 023709 Bucuresti (RO); Topor, Alexandru, Buzau (RO)

(56) References cited:
- RO-A2- 128 603
- SEYED FARID SEYED SHIRAZI ET AL: "A review on powder-based additive manufacturing for tissue engineering: selective laser sintering and inkjet 3D printing", SCIENCE AND TECHNOLOGY OF ADVANCED MATERIALS, vol. 16, no. 3, 20 June 2015 (2015-06-20), page 033502, XP055414960, ISSN: 1468-6996, DOI: 10.1088/1468-6996/16/3/033502

## Description

The present invention relates to the production of three-dimensional structures based on hydroxyapatite and polyurethane-diol by 3D extrusion bioprinting technique, which can be used for the fabrication of ocular implants with interconnected porosity.

A Mexican patent application refers to an ocular intraorbitary implant allowing to fill the space left by the removal of the eyeball of a person, preventing absorption into surrounding tissues and allowing the stability of this cavity in the long term. The invented ocular implant is not porous, facilitating its subsequent removal in situations that require such removal. The implant is made of polymerized bone cement, with zirconium dioxide as contrast material and E141 colorant for better visualization during surgery and monitoring thereof in the early postoperative and also in the long-term [MX 2016005397 A**, 08.06.2016, Spherical**

### intraorbitary implant].

Tissue Engineering is an interdisciplinary field aiming to combine researchers' knowledge about cells, biomaterials, and adequated biochemical factors to create a synthetic structure that allows the growth and regeneration of new tissues [1].

Theoretically, this artificial structure, known as scaffold, should be made of a suitable biomaterial and fabricated to mimic the physical and chemical structure of the host tissue [2,3].

The spongy bone has a porous structure in both volume and surface, with interconnected pores that allow cell migration, vascularization and growth of new tissue [4-6].

In general, fabrication methods of scaffold structures can be grouped into conventional techniques (eg foam, leaching and emulsification) [7] and Additive Layer Manufacturing (ALM) or Additive Manufacturing techniques.

The main drawback of conventional methods is that they do not allow the production of porous scaffolds with complete control of geometric parameters such as pore size, obtaining interconnected pores, material size and porosity [7].

Over the last ten years, ALM has attracted considerable attention from researchers as they allow users to build 3D materials with different complexity levels, which is particularly advantageous when trying to mimic the physical structure of the bone.

To be used for the fabrication of medical scaffolds, the chosen material must fulfill the following conditions: 1) be biocompatible; 2) has a chemically appropriate surface for cell attachment, proliferation and differentiation; 3) be three-dimensional and sufficiently porous, with an interconnected porous network which allows cell growth, nutrients flow and metabolic waste flow; 4) have mechanical properties corresponding to those of the tissue in which it will be implanted [7].

In addition to these conditions, the parameters of the 3D fabrication process must be optimized so that to ensure maximum biocompatibility, osteoconductivity and good mechanical properties.

Depending on the operation principle, the ALM systems were classified into three subgroups: (1) laser based systems, (2) printing systems, and (3) extrusion / nozzle systems [8, 9]. Currently, according to ISO / ASTM52900-15, there are seven categories of ALM processes: Binder Jetting, Directed Energy Deposition, Material Extrusion, Material Jetting, Powder Bed Fusion, Sheet Lamination and Vat Photopolymerization.

### [ISO / ASTM52900-15, Standard Terminology for Additive Manufacturing - General Principles-Terminology, ASTM International, West Conshohocken, PA, 2015, www.astm.org].

Various Rapid Prototyping (RP) techniques, such as selective laser sintering (SLS) [10,11], fused deposition modeling (FDM) [12] and precision extrusion deposition (PED) [13], were used for composite fabrication by combining polymers such as poly(hydroxybutyrate-co-hydroxyvalerate), poly (L-lactide) (PLLA) and polycaprolactone (PCL) with different calcium phosphate phases, such as carbonated hydroxyapatite, hydroxyapatite and β-tricalcium phosphate [14].

Also knows as "Extrusion bioprinting", bioprinting can be defined as a spatial method for layered deposition of a biological material (or support for biological material) based on a computerized, layer-by-layer model using a CAD-CAM system.

The structure of the computerized model can be modified and rearranged with the passage of each layer so as to obtain a complex final model that mimics the tissue structure. The versatility of the technology allows the adoption of an unlimited number of materials combinations for extrusion, thus ensuring the possibility of developing unique three-dimensional models adapted to each case.

*Extrusion bioprinting* technology is a combination of an automated, robot-controlled software and an extrusion distribution system of the material in the customised 3D structure. This process ensures rapidity but also structural integrity due to the computerized continuous deposition system. 3D models can be obtained, generated, imported and exported from any CAD system, including data obtained from medical imaging systems such as computed tomography (CT) and / or Nuclear Magnetic Resonance (NMR). Unlike other manufacturing processes, extrusion bioprinting does not require large amounts of energy and is an environmentally friendly method.

The 3D printing technique (SFF) has been used to make 3D scaffold structures based on hydroxyapatite and various organic polymers such as poly (L-lactide-co-glycolide) (PLGA) copolymer [15], polyvinyl alcohol (PVA), Collagen [16], PEO / PBT commercial polymer [14]. Combinations requiring the use of organic solvents as a binder, e.g. PLGA and β-TCP bound to chloroform [15], present an intrinsic disadvantage because there is always the risk of finding toxic solvent residues in the 3D structure [7].

In order to obtain as rigid and robust calcium phosphate structures by three dimensional printing, citric acid, phosphoric acid, tartaric acid binders were used, but this method proved to be disadvantageous for tissue engineering. Zhou et al. [17] used a mixture of hydroxyapatite with calcium sulphate, HAp: CaSO₄ ratio = 25:75 (weight percent) for scaffolds fabrication by three dimensional printing. In a recent study, J. Inzana et al. [16], obtained a composite scaffold based on calcium phosphate and collagen, by 3D printing method at low temperatures.

For this purpose, 5-20% phosphoric acid was used as binder and Tween 80 was added as a non-cytotoxic surfactant in the proportion of 0.25% in the binder solution. The optimal concentration of the binder solution (8.75%) for which the cytocompatibility and mechanical strength are maximized and the required amount of surfactant (Tween 80) for improved printing have been established. Also, collagen has previously been dissolved in the binder solution to further enhance the fabrication of calcium phosphate -collagen composites by 3D printing. In 2013, A. Nandakumar et al. [14] reported the production of two types of polymer-hydroxyapatite composite scaffold by 3D deposition using a Bioplotter. PolyActive ™ (PA), a commercial copolymer of poly (ethylene oxide-terephthalate) / poly (butylene terephthalate) (PEO / PBT) was used.

In the first embodiment, polymer-ceramic composite filaments with the desired mass ratio (maximum 15% HAp) were extruded. They were used as material for scaffold fabrication using Bioplotter. In the second route, polymer scaffolds were obtained by 3D deposition, while the ceramic particles were fabricated in the form of columns by sintering the ceramic paste with the use of stereolithography. The two components were then manually assembled by pressing HAp into the pores of the polymer scaffold, thus creating the composite material. This method of 3D deposition using Bioplotter allows scaffolds fabrication with controlled porosity, pore size, interconnectivity and fiber orientation between successive layers (at 45 or 90 degrees). The method has been successfully used for the fabrication of polymer scaffolds for tissue engineering (cartilage and osteocartilaginous defects). [14, 18-19]. Although it is a very versatile technique, the fabrication of polymer-ceramic composite scaffolds is difficult due to the high viscosity of the polymer-ceramic paste, which can lead to clogging of the nozzles. In addition to affecting fabrication and processability, these phenomena limit the amount of ceramic that can be incorporated into the scaffold, although this ceramic determines the osteoconductivity and osteoinductivity of composite materials.

Recently, a biological ceramic material for bone repair, produced by 3D printing method from β-TCP, hydroxyapatite and polylactic acid (PLA) with a molecular weight of 100,000-120,000, viscosity between 0,79 dL/g -0,84 dL/g, porosity of the three-dimensional structure 70% -90%, and the side of a square that forms the grid between 100 and 300 micrometers, has been patented [CN105770988 (A**) - Bone repairing biological ceramic material based on 3D printing and preparation method thereof, 20.07.2016**].

Another invention relates to the synthesis of a printable hybrid material based on carboxymethyl chitosan (CMC) and polyphosphate (polyP). The two polymers are bound by calcium ions. CMC-PolyP in combination with alginate is biocompatible, biodegradable and useful for 3D printing and 3D cell printing (bioprinting) [WO 2016/012583**, Printable morphogenetic phase-specific chitosan-calcium polyphosphate scaffold for bone repair**]**.** Seyed Farid Seyed Shirazi et al. (Science and Technology of Advanced Materials; vol. 16, no. 3, 20 June 2015, page 033502) discloses 3D printing methods for tissue engineering using composite powders of hydroxyapatite and a polymer.

In contrast to the above-described compositions for depositing scaffold structures by different 3D printing techniques, in which the hydroxyapatite and the organic polymer are synthesized separately or commercially available, and then mixed with a binder to obtain the extrusion printing paste, the present invention relates to obtaining of three-dimensional structures from nanostructured hybrid powders based on hydroxyapatite and polyurethane diol synthesized in situ in hydrothermal conditions under high pressure (1000 bar) and low temperatures (100 °C), and spray dried.

The technical problem solved by the present invention relates to the provision of hybrid nanostructured powders based on hydroxyapatite and polyurethane-diol, which have a *homogeneous chemical structure* (hydroxyapatite in the hybrid structure respects the specific molar ratio Ca: P = 1.67 of HAp in the bone mineral structure) and *controlled morphology* (spherical particles, which ensure the processing of high viscosity pastes without clogging the 3D print nozzles). The advantages of hydrothermal synthesis at high pressure and low temperatures consist of:
i) low energy consumption by applying very high pressure (the energy consumed to increase the temperature by 5 units is equal to that required to increase the pressure by 4000 units in the system);
ii) the negative value of ΔV (ΔV = Σ (V / Z) (j) - Σ (V / Z) (i) where i = reactant and j = reaction product; shifting equilibrium to compounds with the smallest volume.
iii) improving chemical reactivity;
iv) single-step synthesis of nanocrystalline materials without the need for further heat treatment;
v) retaining the unaltered structure of the polymer due to the low reaction temperature (100 °C);
vi) environment-friendly technology, in a closed autoclave, without the release of toxic compounds.

The present invention relates to the production of three-dimensional structures based on hydroxyapatite and polyurethane-diol by 3D extrusion bioprinting at room temperature using commercial water-soluble polymer binders. The 3D structures thus obtained can be used for the fabrication of ocular implants with interconnected porosity required for their vascularization.

By applying the invention, the drawbacks of the materials previously used to make the paste are overcome in that the proposed materials consist of hydroxyapatite and polyurethane diol in a weight ratio of 4: 1, and the particles are spherical having a diameter of. 0.5-8 µm. Because of this weight ratio and commercial polymers used as binders, the total amount of hydroxyapatite incorporated into the scaffold is not so great as to cause clogging of the nozzles. The incorporation of the polyurethane during synthesis and spray drying aids the extrusion of the paste and the formation of a rigid 3D structure with the required hardness for medical application (ocular implant fixed with titanium screws).

The attached figures show the SEM micrographs of a hybrid powder based on hydroxyapatite and polyurethane-diol having a 4: 1 weight ratio.

To realize 3D structures according to the invention, commercial polyurethane-diol may be used in the hydrothermal synthesis of hybrid powders. The process of synthesizing hybrid nanostructured powders takes place under hydrothermal conditions at temperatures between 25 and 120 °C (preferably 80-100 °C) and pressures in the range of 1000-3000 bar (preferably 1000-2000 bar) . The hybrid material thus obtained is spray dried at temperatures between 100 and 300 °C (preferably 100-150 °C). The resulting powder is mechanically mixed with water soluble polymer binders (Mowiflex and Baymedix FD 103) in well defined proportions to obtain a paste which can be further used for 3D extrusion bioprinting.

Two examples of embodiments of the invention are set forth below without limiting the use of this process in the proposed technical field:
**Example 1.** Mix 5 g of hybrid nanostructured powder based on hydroxyapatite, HAp and polyurethane diol, PU (weight ratio HAp: PU = 4: 1) with 8.8 ml Mowiflex, 20% solution and 0.5 ml Baymedix FD 103, solution 57%. Apply on a plexiglass support using a 3D BioScaffolder system, a 2 cm side cube using a nozzle 0.4 mm in diameter, 31 mm nozzle length and 80% nozzle thickness. The angle of rotation between two successive layers is 90 ° and the distance between the extruded yarns is 1.3 mm. Movement speed of the deposition head is 400 mm / min.
**Example 2.** Mix 5 g of hybrid nanostructured powder based on hydroxyapatite, HAp and polyurethane diol, PU (weight ratio HAp: PU = 4: 1) with 8.8 ml Mowiflex, 20% solution. Apply on a plexiglass support using a 3D BioScaffolder system a 2.5 cm side cube using a 0.8 mm diameter nozzle, 31 mm nozzle length and 80% layer thickness of the nozzle diameter. The angle of rotation between two successive layers is 90 ° and the distance between the extruded yarns is 1.3 mm. Movement speed of the deposition head is 400 mm / min.

### Bibliography

1. J.D. Sipe, Reparative medicine: growing tissues and organs, 961 (2002) 1-9.
2. Q. Chen, A. Boccaccini, H. Zhang, D. Wang, M. Edirisinghe, J. Am. Ceram. Soc. 89 (2006) 1534-1539.
3. S. C. Cox, J. A. Thornby, G. J. Gibbons, M. A.Williams, K. K. Mallick, Materials Science and Engineering C 47 (2015) 237-247.
4. R.Z. Legeros, S. Lin, R. Rohanizadeh, D.Mijares, J.P. Legeros, J. Mater. Sci.Mater. Med. 14 (2003) 201-209.
5. A.G. Mikos, G. Sarakinos, M.D. Lyman, D.E. Ingber, J.P. Vacanti, R. Langer, Biotechnol. Bioeng. 42 (1993) 716-723.
6. D.J. Mooney, D.F. Baldwin, N.P. Suh, J.P. Vacanti, R. Langer, Biomaterials 17 (1996) 1417-1422.
7. A. Butscher, M. Bohner, S. Hofmann, L. Gauckler, R. Müller, Acta Biomaterialia 7 (2011) 907-920.
8. S.J. Hollister, Nat. Mater. 4 (2005) 518-524.
9. S.M. Giannitelli, P. Mozetic, M. Trombetta, A. Rainer, Acta Biomaterialia 24 (2015) 1-11.
10. [Duan B, Wang M, Zhou WY, Cheung WL, Li ZY, Lu WW. Acta Biomater 2010;6:4495-505.
11. Tan KH, Chua CK, Leong KF, Cheah CM, Cheang P, Abu Bakar MS, et al. Biomaterials 2003;24:3115-23.
12. Yefang Z, Hutmacher DW, Varawan SL, Meng LT. Int J Oral Max Surg 2007;36:137-45.
13. Shor L, Guceri S, Wen XJ, Gandhi M, Sun W. Biomaterials 2007;28:5291-7.
14. A. Nandakumar, C. Cruz, A. Mentink, Z. T. Birgani, L.Moroni, C. van Blitterswijk, P. Habibovic, Acta Biomaterialia 9 (2013) 5708-5717.
15. T.D. Roy, J.L. Simon, J.L. Ricci, E.D. Rekow, V.P. Thompson, J.R. Parsons, J. Biomed.Mater. Res. A 66A (2003) 283-291.
16. J.A. Inzana, D. Olvera, S. M. Fuller, J. P. Kelly, O. A. Graeve, E.M. Schwarz, S. L. Kates, H. A. Awad, Biomaterials 35 (2014) 4026-4034.
17. Z. Zhou, F.Buchanan, C. Mitchell, N. Dunne, Materials Science and Engineering C 38 (2014) 1-10.
18. Woodfield TBF, Malda J, de Wijn J, Peters F, Riesle J, van Blitterswijk CA. Biomaterials 2004;25:4149-61.
19. Moroni L, Hamann D, Paoluzzi L, Pieper J, de Wijn JR, van Blitterswijk CA.PLoS One 2008;3:e3032.

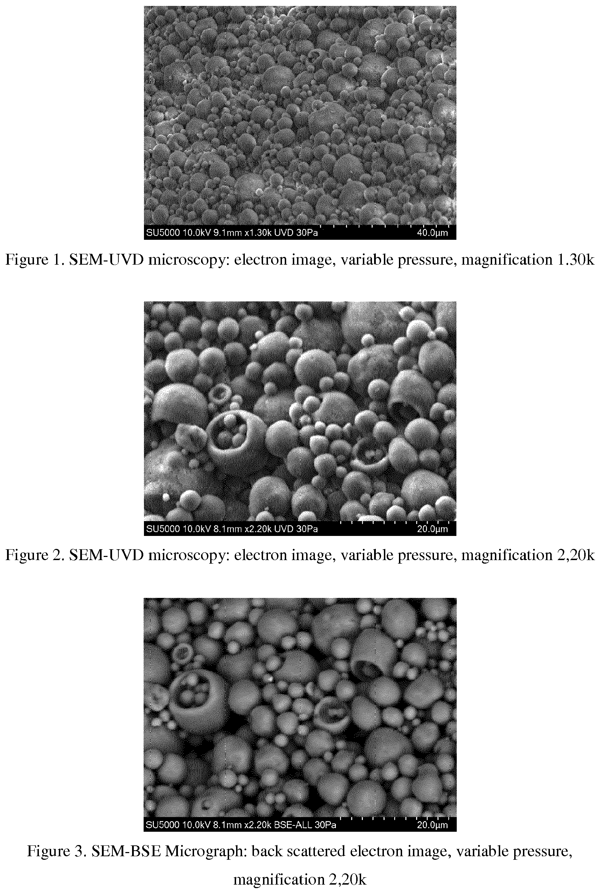

## Claims

1. 3D structures for ocular implants with interconnected porosity made by the 3D extrusion bioprinting method, **characterized in that** they are obtained from spray dried hybrid powder based on hydroxyapatite and polyurethane diol synthesized by the hydrothermal process at a pressure p = 1000 bar and temperature t = 100 °C, in 4: 1 weight ratio, in the form of spherical particles, having a diameter of 0.5-8 µm; mixed with water-soluble polymer binders.

## Patentansprüche

1. 3D-Strukturen für Augenimplantate mit interkonnektierender Porosität, durch das 3D-Extrusions-Biodruckverfahren hergestellt, **dadurch gekennzeichnet, dass** sie aus sprühgetrocknetem Hybridpulver basierend auf Hydroxylapatit und Polyurethandiol erhalten werden, das durch den hydrothermalen Prozess bei einem Druck p = 1000 bar und einer Temperatur t = 100 °C im Gewichtsverhältnis 4:1 in Form von kugelförmigen Teilchen mit einem Durchmesser von 0,5 bis 8 µm hergestellt wird; gemischt mit wasserlöslichen Polymerbindemitteln.

## Revendications

1. Les structures 3D pour les implants oculaires à porosité interconnectée réalisées à l'aide de la méthode de bio-impression par extrusion 3D, **caractérisées en ce qu'**elles sont obtenues à partir de la poudre hybride séchée par pulvérisation à base d'hydroxyapatite et de polyuréthane diol synthétisés par le procédé hydrothermal à une pression p = 1000 bars et à une température t = 100 °C, dans le rapport pondéral 4 : 1, sous forme de particules sphériques, ayant un diamètre de 0,5-8 µm ; en combinaison avec des liants polymères solubles dans l'eau.
